(19) Europäisches Patentamt
European Patent Office
Office européen des brevets

(11) EP 2 102 141 B1

(12) **EUROPÄISCHE PATENTSCHRIFT**

(45) Veröffentlichungstag und Bekanntmachung des Hinweises auf die Patenterteilung:
**01.04.2015 Patentblatt 2015/14**

(21) Anmeldenummer: **06793086.7**

(22) Anmeldetag: **31.08.2006**

(51) Int Cl.:
***C07C 51/41*** (2006.01) ***C07C 57/04*** (2006.01)
***C08F 220/06*** (2006.01) ***B01J 19/24*** (2006.01)
***B01J 19/00*** (2006.01)

(86) Internationale Anmeldenummer:
**PCT/EP2006/065842**

(87) Internationale Veröffentlichungsnummer:
**WO 2007/028746 (15.03.2007 Gazette 2007/11)**

(54) **NEUTRALISATIONSVERFAHREN**

NEUTRALIZATION METHOD

PROCEDE DE NEUTRALISATION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **07.09.2005 DE 102005042605**

(43) Veröffentlichungstag der Anmeldung:
**23.09.2009 Patentblatt 2009/39**

(73) Patentinhaber: **BASF SE**
**67056 Ludwigshafen (DE)**

(72) Erfinder:
- **WEISMANTEL, Matthias**
  **63637 Jossgrund (DE)**
- **CLAESSENS, Louis**
  **B-2950 Kapellen (BE)**
- **POSSEMIERS, Karl, J.**
  **B-2900 Schoten (BE)**
- **DE KAEY, Ronny**
  **B-2531 Vremde (BE)**
- **FUNK, Rüdiger**
  **65527 Niedernhausen (DE)**

(56) Entgegenhaltungen:
**EP-A- 1 470 905** **EP-A2- 0 372 706**
**WO-A-03/095410** **WO-A1-2005/073260**
**JP-A- H01 135 761** **JP-A- H11 349 555**
**JP-A- 2000 053 641**

- **"Fluka Chemikalienkatalog", Fluka page 51,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Neutralisationsverfahren für ethylenisch ungesättigte Carbonsäuren, wobei mindestens ein Stoffstrom der Neutralisation kontinuierlich bestimmt wird, sowie eine Vorrichtung zur Durchführung des Verfahrens.

Weitere Ausführungsformen der vorliegenden Erfindung sind den Ansprüchen und der Beschreibung zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure bevorzugt werden. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung der wasserabsorbierenden Polymere wird beispielsweise in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seiten 73 bis 103, beschrieben. Das bevorzugte Herstellungsverfahren ist die Lösungs- oder Gelpolymerisation. Bei dieser Technologie wird zunächst eine Monomermischung hergestellt, die diskontinuierlich neutralisiert und dann in einen Polymerisationsreaktor überführt wird, oder bereits im Polymerisationsreaktor vorgelegt wird. Im sich anschließenden diskontinuierlichen oder kontinuierlichen Verfahren erfolgt die Reaktion zum Polymergel, das im Falle einer gerührten Polymerisation bereits zerkleinert wird. Das Polymergel wird anschließend getrocknet, gemahlen und gesiebt und dann zur weiteren Oberflächenbehandlung transferiert.

Ein kontinuierliches Polymerisationsverfahren liegt beispielsweise der WO 01/38402 zugrunde, wobei die wässrige Monomerlösung kontinuierlich einem Mischkneter mit mindestens zwei achsparallel rotierenden Wellen zugeführt wird.

Kontinuierliche Gelpolymerisationen sind weiterhin bekannt aus WO 03/004237, WO 03/022896 und WO 01/016197.

Sowohl bei der kontinuierlichen als auch bei der diskontinuierlichen Polymerisation wird die Acrylsäure im Falle der Vorneutralisation diskontinuierlich neutralisiert. Typischerweise werden im Polymerisationsreaktor im Falle der Lösungspolymerisation die Edukte Acrylsäure, Wasser, optionale Comonomere und Natronlauge diskontinuierlich dosiert und vermischt. In diesem Schritt wird weitestgehend der restliche Polymerisationsverlauf und auch die zu erwartenden Polymereigenschaften festgelegt. Der Vernetzungsgrad des Basispolymers sowie der Neutralisationsgrad werden typischerweise in diesem Schritt bestimmt. Der Neutralisationsgrad der Monomeren liegt zwischen 0 und 80 mol-%. Im Falle der sauren Polymerisation wird das erhaltene Polymergel üblicherweise zu 50 bis 80 mol-%, vorzugsweise zu 60 bis 75 mol-% nachneutralisiert, indem Natriumhydroxid- oder Natriumcarbonatlösung zum sauren Polymergel zugegeben und eingearbeitet wird.

Neutralisationsverfahren werden beispielsweise in EP-A 0 372 706, EP-A 0 574 260 und WO 03/051415 beschrieben.

EP-A 0 372 706 beschreibt ein dreistufiges Neutralisationsverfahren, bei dem in einer ersten Stufe Acrylsäure und Natronlauge gleichzeitig dosiert werden, so dass ein Neutralisationsgrad von 75 bis 100 mol-% eingehalten wird, in einer zweiten Stufe der Neutralisationsgrad auf 100,1 bis 110 mol-% angehoben wird um in der eingesetzten Acrylsäure als Verunreinigung enthaltene Diacrylsäure zu hydrolysieren, und in einer dritten Stufe durch Zusatz von weiterer Acrylsäure ein Neutralisationsgrad von 20 bis 100 mol-% eingestellt wird.

EP-A 0 574 260 offenbart auf Seite 7, Zeilen 38 bis 41, dass bei der Neutralisation vorteilhaft Natronlauge vorgelegt und anschließend Acrylsäure unter Kühlung zugesetzt wird.

WO 03/051415 lehrt ein Verfahren zur Herstellung wasserabsorbierender Polymere, bei dem die Monomerlösung eine Mindesttemperatur von 40°C aufweist.

WO 03/095410 A1 beschreibt ein Destillationsverfahren für Acrylsäure, wobei der Brüden unmittelbar aus der Gasphase in einer Alkalilösung aufgenommen wird.

Es ist bekannt, dass sich die Reaktivität der Acrylsäure sehr stark von der ihrer Salze unterscheidet, weshalb auch der Polymerisationsverlauf stark abhängig ist vom pH-Wert, bei dem er stattfindet. In einer anschaulichen Darstellung in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seite 35, ist die Polymerisationsgeschwindigkeit als Funktion des pH-Wertes aufgetragen. Demnach durchläuft die Polymerisationsgeschwindigkeit ein Minimum bei einem pH-Wert von 6 bis 7. Dies entspricht allerdings dem pH-Wert, der in aller Regel bei den Verkaufsprodukten gewünscht ist. Erklärt wird dieses Verhalten dadurch, dass es zwischen dem geladenen Monomeren in

Salzform und der wachsenden Radikalkette zu elektrostatischen Abstoßungsreaktionen kommt, die im Falle der weitestgehend undissoziierten Acrylsäure nicht vorliegen, was zur Verlangsamung des Reaktionsverlaufs führt.

Es ist auch bekannt, dass unneutralisierte Acrylsäure leichter polymerisiert werden kann als vorneutralisierte Systeme. Diese Differenz wird jedoch bei steigender Monomerkonzentration verringert, vor allem deswegen, weil eine höhere Monomerkonzentration die Dissoziation der Acrylsäuresalze unterdrückt.

Um all diesen reaktionstechnischen Details Rechnung zu tragen, werden bei der Reaktionsführung üblicherweise Kompromisse eingegangen.

Generell wird der Neutralisationsgrad der Acrylsäure bereits vor dem Eintritt in die kontinuierliche Polymerisation eingestellt. Die Neutralisation erfolgt diskontinuierlich. Die diskontinuierliche Neutralisation hat den Vorteil, dass die Dosierung von Acrylsäure und/oder Natronlauge temperaturgeregelt erfolgen kann. Damit werden Überhitzungen und unerwünschte Polymerisationen in dem Ansatzbehälter vermieden. Der Neutralisationsgrad wird entsprechend den Polymerisationsbedingungen und dem erwünschten Absorptionsprofil gewählt und wahlweise in einer nachgeschalteten Neutralisation, die zumeist am Polymergel erfolgt, korrigiert.

Bei der Neutralisation werden Neutralisationsgrad und Feststoffgehalt der neutralisierten Lösung auf den gewünschten Wert eingestellt. Dies geschieht üblicherweise über die Mengenverhältnisse der Einsatzstoffe, d.h. eine mengengesteuerte Dosierung von beispielsweise Acrylsäure, Natronlauge und Wasser.

Problematisch bei der üblichen Mengendosierung ist, dass mögliche Konzentrationsabweichungen der Einsatzstoffe unberücksichtigt bleiben.

Der Gehalt handelsüblicher 50gew.-%iger Natronlauge an Natriumhydroxid ist beispielsweise zu 50 +/- 0,5 Gew.-% spezifiziert. Dies bedeutet, dass eine teilneutralisierte Acrylsäure mit einem Neutralisationsgrad von 75 % einen Neutralisationsgrad von 75 +/- 0,75 % aufweisen kann. Wird diese Lösung zur Herstellung wasserabsorbierender Polymere verwendet, so wird diese Abweichung bis in das Endprodukt übertragen, inklusive möglicher Abweichungen von den gewünschten Polymereigenschaften.

Werden die Einsatzstoffe der Neutralisation über ein zentrales Rohrleitungssystem bezogen, so besteht die Möglichkeit, dass die Konzentration der Einsatzstoffe durch im Leitungssystem vorhandenes Restwasser verändert wird. Aus Sicherheitsgründen wird das Leitungssystem vor Wartungs- und Instandhaltungsmaßnahmen mit Wasser gespült. Dieses Wasser kann üblicherweise anschließend nicht vollständig entfernt werden und verbleibt als Restwasser im Leitungssystem.

Aufgabe der vorliegenden Erfindung war die Bereitstellung eines Neutralisationsverfahrens, wobei auftretende Konzentrationsabweichungen der Einsatzstoffe erkannt und, vorzugsweise automatisch, korrigiert werden.

Gelöst wurde die Aufgabe durch ein Neutralisationsverfahren, wobei mindestens eine ethylenisch ungesättigte Carbonsäure zumindest teilweise mit einer Base neutralisiert wird, die Temperatur der ethylenisch ungesättigten Carbonsäure von 15 bis 40°C beträgt und die ethylenisch ungesättigte Carbonsäure Acrylsäure ist, dadurch gekennzeichnet, dass der Gehalt mindestens einer Komponente mindestens eines Stoffstromes der Neutralisation kontinuierlich ermittelt und zur Regelung der Neutralisation genutzt wird.

Mindestens der Gehalt einer Komponente des Stoffstromes wird ermittelt beispielsweise der Gehalt an Natriumhydroxid in der eingesetzten Natronlauge oder der Gehalt an Acrylsäure und Natriumacrylat in der neutralisierten Lösung. Dabei kann der Gehalt der gewünschten Komponente direkt oder indirekt ermittelt werden.

Ein mögliches direktes Verfahren ist beispielsweise die NIR-Spektroskopie. Aus den erhaltenen NIR-Spektren kann anhand von geeigneten Eichkurven direkt die Konzentration der Komponenten bestimmt werden.

Vorzugsweise wird mittels der NIR-Spektroskopie der Gehalt an ethylenisch ungesättigter Carbonsäure und deren Salzen in der neutralisierten Lösung bestimmt. Diese Daten sind dann Grundlage zur Bestimmung von Neutralisationsgrad und Feststoffgehalt der neutralisierten Lösung.

Indirekte Analysenverfahren werten Messwerte aus, die für sich allein noch keine Rückschlüsse auf Konzentrationen zulassen. Ein klassisches indirektes Verfahren ist die Regelung von Destillationskolonnen über Temperaturen. Die Temperaturen an sich geben noch keinen Hinweis auf Konzentrationen. Erst die Kenntnis der Siedediagramme eines zu trennenden Zweistoffgemisches ermöglicht eine eindeutige Zuordnung von Temperatur und Konzentration.

Ein mögliches indirektes Verfahren ist beispielsweise die Bestimmung des Alkaligehaltes in wässrigem Alkali über die Dichte und die Temperatur. Beispielsweise gilt für den Gehalt an Natriumhydroxid in ca. 50gew.-%iger Natronlauge die Beziehung

$$c\,[\text{Gew.-\%}] = (0{,}10657 \times \sigma\,[\text{g/cm}^3]) + (0{,}07016 \times T\,[°\text{C}]) - 114$$

wobei c der Gehalt an Natriumhydroxid, $\sigma$ die Dichte der Natronlauge und T die Temperatur der Natronlauge bedeuten.

Kontinuierlich bedeutet, dass in regelmäßigen Intervallen ein Messwert erzeugt wird, üblicherweise mindestens 1 mal pro 10 Minuten, vorzugsweise mindestens 1 mal pro Minute, besonders bevorzugt mindestens 1 mal pro 10

Sekunden, ganz besonders bevorzugt 1 mal pro Sekunde. Kürzere Intervalle sind möglich.

Die durch kontinuierliche Analysen erhaltenen Messwerte werden zur Regelung der Neutralisation genutzt, vorzugsweise unter Verwendung eines handelsüblichen Prozeßleitsystems.

Der Neutralisationsgrad wird über das Verhältnis von Acrylsäure zu Natronlauge und der Feststoffgehalt über das Verhältnis von (Acrylsäure+Natronlauge) zu Wasser festgelegt.

Wird beispielsweise ein zu hoher Neutralisationsgrad ermittelt, so wird die Dosierung der Natronlauge entsprechend vermindert und die Dosierung von Wasser entsprechend erhöht.

Wird beispielsweise ein zu hoher Feststoffgehalt ermittelt, so wird die Dosierung der Natronlauge und Acrylsäure entsprechend vermindert und die Dosierung von Wasser entsprechend erhöht.

Wird beispielsweise der Gehalt an Natronlauge der verwendeten 50 gew.-%igen Natronlauge zu 52 Gew.-% ermittelt, so wird die Dosierung der Natronlauge entsprechend vermindert und die Dosierung von Wasser entsprechend erhöht.

Die Temperatur der ethylenisch ungesättigte Carbonsäure beträgt üblicherweise von 15 bis 40°C, vorzugsweise bis 35°C, besonders bevorzugt bis 30°C, ganz besonders bevorzugt von 15 bis 25°C, wobei auf einen ausreichenden Abstand zu Schmelzpunkt zu achten ist. Bei Verwendung von Acrylsäure sollte eine Temperatur von 15°C auf keinen Fall unterschritten werden.

Als Base ist wässriges Alkali bevorzugt. Wässriges Alkali sind alle alkalisch reagierenden wässrigen Lösungen, d.h. wässrige Lösungen mit einem pH-Wert von mindestens 8, vorzugsweise mindestens 10, besonders bevorzugt mindestens 12, ganz besonders bevorzugt mindestens 14.

Die im wässrigen Neutralisationsmittel einsetzbaren alkalischen Salze sind vorzugsweise Alkalimetallhydroxide, Alkalimetalloxide, Alkalimetallcarbonate und Alkalimetallhydrogencarbonate sowie deren Mischungen. Statt Alkalimetallsalzen können auch Ammoniumsalze verwendet werden. Natrium und Kalium als Alkalimetalle sind besonders bevorzugt, ganz besonders bevorzugt jedoch Natriumhydroxid, Natriumcarbonat oder Natriumhydrogencarbonat sowie deren Mischungen. Üblicherweise beträgt der Alkalgehalt im wässrigen Alkali mindestens 10 Gew.-%, vorzugsweise mindestens 20 Gew.-%, bevorzugt mindestens 30 Gew.-%, ganz besonders bevorzugt mindestens 40 Gew.-%.

Die Temperatur des wässrigen Alkali beträgt üblicherweise von 0 bis 45°C, vorzugsweise von 5 bis 40°C, besonders bevorzugt von 10 bis 35°C, ganz besonders bevorzugt von 15 bis 30°C, wobei Übersättigungen und damit Ausfällungen zu vermeiden sind.

Beträgt der Alkaligehalt des wässrigen Alkali mindestens 25 Gew.-%, so sind höhere Temperaturen vorteilhaft, üblicherweise von 10 bis 60°C, vorzugsweise von 20 bis 55°C, besonders bevorzugt von 30 bis 50°C, ganz besonders bevorzugt von 40 bis 45°C.

Das Verhältnis von ethylenisch ungesättigter Carbonsäure zu Base wird üblicherweise so gewählt, dass der Neutralisationsgrad der ethylenisch ungesättigter Carbonsäure nach der Neutralisation vorzugsweise von 25 bis 85 mol-%, bevorzugt von 27 bis 80 mol-%, besonders bevorzugt von 27 bis 30 mol-% oder 40 bis 75 mol-%, beträgt.

Der Neutralisationsgrad ist das Molverhältnis von neutralisierter ethylenisch ungesättigter Carbonsäure nach der Neutralisation zur Gesamtmenge eingesetzter ethylenisch ungesättigter Carbonsäure vor der Neutralisation.

Die Neutralisation wird vorzugsweise kontinuierlich durchgeführt. Dies bedeutet, dass dem Neutralisationsbereich ethylenisch ungesättigte Carbonsäure und/oder Base zugeführt wird und dem Neutralisationsbereich gleichzeitig neutralisierte Lösung entnommen wird. An- und Abfahrvorgänge des kontinuierlichen Neutralisationsverfahrens sind hiervon selbstverständlich ausgenommen.

Vorzugsweise wird die dem Neutralisationsbereich kontinuierlich entnommene Lösung zumindest teilweise kontinuierlich in die Polymerisation überführt.

Die Polymerisation wird vorzugsweise ebenfalls kontinuierlich durchgeführt.

Der Neutralisationsbereich ist der Bereich, in dem die Neutralisation weitgehend stattfindet, d.h. der Bereich in dem ethylenisch ungesättigte Carbonsäure und Base unter Salzbildung reagieren (Neutralisation).

Die Neutralisation ist weitgehend abgeschlossen, wenn der Umsatz der Neutralisation mindestens 90 mol-%, vorzugsweise mindestens 95 mol-%, bevorzugt mindestens 98 mol-%, ganz besonders bevorzugt mindestens 99 mol-%, beträgt. Der Umsatz kann leicht über die freigesetzte Neutralisationswärme durch Vergleich mit der theoretischen Wärmetönung ermittelt werden.

Die vorzugsweise kontinuierliche Neutralisation wird so durchgeführt, dass die Temperatur der neutralisierten Lösung vorzugsweise weniger als 60°C, bevorzugt weniger als 50°C, besonders bevorzugt weniger als 40°C, ganz besonders bevorzugt weniger als 30°C, beträgt, wobei die Temperatur die Durchschnittstemperatur nach der Neutralisation ist, d.h. die Mitteltemperatur nach vollständiger Wärmetönung.

Zusätzlich kann die neutralisierte Lösung mit Wasser verdünnt werden. Über die Verdünnung mit Wasser kann der Feststoffgehalt der neutralisierten Lösung eingestellt werden. Der Feststoffgehalt ist die Summe der Gewichtanteile an neutralisierter ethylenisch ungesättigter Carbonsäure und wahlweise überschüssiger ethylenisch ungesättigter Carbonsäure oder überschüssiger Base. Der Feststoffgehalt der neutralisierten Lösung beträgt typischerweise von 10 bis 80 Gew.-%, vorzugsweise von 20 bis 70 Gew.-%, besonders bevorzugt von 30 bis 60 Gew.-%.

Die Temperatur des Wassers beträgt üblicherweise von über 0 bis 40°C, vorzugsweise von 5 bis 35°C, besonders bevorzugt von 10 bis 30°C, ganz besonders bevorzugt von 15 bis 25°C.

Vorteilhaft wird die neutralisierte Lösung gekühlt, wobei die zur Kühlung einsetzbaren Wärmeaustauscher keiner Beschränkung unterliegen. Die neutralisierte Lösung wird auf eine Temperatur von vorzugsweise weniger als 50°C, bevorzugt weniger als 40°C, besonders bevorzugt weniger als 30°C, ganz besonders bevorzugt weniger als 20°C, gekühlt. Die Kühlung sollte möglichst nah an der Neutralisation sein, da so hohe Verweilzeiten der neutralisierten Lösung bei hohen Temperaturen vermieden werden können.

Vorzugsweise werden Wasser und Base vorgemischt. In diesem Fall kann die dabei freiwerdende Lösungswärme bereits vor der Neutralisation, beispielsweise mittels geeigneter Wärmeaustauscher, abgeführt werden.

In einer besonders bevorzugten Ausführungsform der vorliegenden Erfindung wird ein Teil der neutralisierten Lösung in die Neutralisation rückgeführt, vorzugsweise gekühlt.

Durch die Rückführung kann die Neutralisationswärme und die Lösungswärme besser verteilt und Temperaturspitzen (Peaktemperatur) in der Mischung niedrig gehalten werden. Der Anteil rückgeführter neutralisierter Lösung beträgt üblicherweise von 25 bis 99%, vorzugsweise von 33 bis 98%, besonders bevorzugt von 50 bis 95%, ganz besonders bevorzugt von 80 bis 90%, jeweils bezogen auf die neutralisierte Lösung.

Die ethylenisch ungesättigte Carbonsäure, die Base und wahlweise das Wasser können an beliebigen Stellen in die rückgeführte neutralisierte Lösung dosiert werden. Vorzugsweise werden die Flüssigkeiten nacheinander dosiert, besonders bevorzugt Base und ethylenisch ungesättigte Carbonsäure nacheinander, ganz besonders bevorzugt Wasser, Base und ethylenisch ungesättigte Carbonsäure nacheinander.

Vorteilhaft wird mindestens eines der Edukte über zwei oder mehr getrennte Zugabestellen dosiert.

Beispielsweise können die Edukte über zwei, drei, vier, fünf oder sechs Zugabestellen dosiert werden, wobei die Zugabestellen vorzugsweise so angeordnet sind, dass sie eine gemeinsame Achse aufweisen (für zwei Zugabestellen) oder einen symmetrischen Stern bilden (für mindestens drei Zugabestellen) und die Achse bzw. Stern senkrecht zur Flussrichtung der neutralisierten Lösung befindet (Mehrfachzugabestellen).

Besonders vorteilhaft wird die Base dosiert, wenn zwei, drei oder vier Mehrfachzugabestellen hintereinander angeordnet werden.

Das Aufteilen in mehrere Zugabestellen bewirkt eine gleichmäßigere Durchmischung und niedrigere Temperaturspitzen, was die Gefahr unerwünschter Polymerisation vermindert.

In einer weiteren Ausführungsform werden Wasser und Base so dosiert, dass das Wasser die Base beim Eintritt in die Neutralisation umhüllt. Dazu können beispielsweise zwei ineinander gesteckte Rohre verwendet werden, wobei die Base über das Innenrohr und das Wasser über den Ringspalt zwischen innerem und äußeren Rohr dosiert werden.

Vorteilhaft enthält die Neutralisation einen zusätzlichen Behälter als Puffergefäß.

Eine beispielhafte erfindungsgemäße Neutralisation zeigt Figur 1, wobei die Bezugszeichen folgende Bedeutungen haben:

| | |
|---|---|
| $Z_1$ bis $Z_3$ | Zuführungen für Edukte 1 bis 3 |
| A | Ableitung |
| B | Behälter |
| $M_1$ und $M_2$ | Messgeräte 1 und 2 |
| P | Pumpe |
| R | Ringleitung |
| W | Wärmeaustauscher |

Mittels einer Pumpe P wird neutralisierte Lösung teilweise über die Ringleitung R rückgeführt. Der Rest der neutralisierten Lösung wird über die Ableitung A der weiteren Verwendung zugeführt. Der Behälter B dient als Puffer. Über die Zuleitung $Z_1$ wird vorzugsweise 50gew.-%ige Natronlauge, über die Zuleitung $Z_2$ wird vorzugsweise Acrylsäure und über die Zuleitung $Z_3$ wird vorzugsweise Wasser dosiert.

Die Messgeräte $M_1$ und/oder $M_2$ erzeugen die zur Analyse notwendigen Messwerte. In dem erfindungsgemäßen Verfahren wird üblicherweise das Messgerät $M_1$ allein, das Messgerät $M_2$ allein oder beide eingesetzt.

Damit die Edukte möglichst intensiv in die rückgeführte neutralisierte Lösung eingemischt werden, sollte die Strömung an der Einmischstelle möglichst turbulent sein. Die Einmischstelle ist der Ort, wo das jeweilige Edukt auf die rückgeführte neutralisierte Lösung trifft.

In einer bevorzugten Ausführungsform der vorliegenden Erfindung wird mindestens eines der Edukte in ein, vorzugsweise werden alle Edukte in ein, besonders bevorzugt werden alle Edukte in ein gemeinsames, Venturi-Rohr dosiert.

Ein Venturi-Rohr ist eine in ihrer Länge begrenzte Rohrverengung, in der Druckverlust weitgehend reversibel in kinetische Energie umgewandelt wird. Dazu wird der Querschnitt $F_1$ auf der Strecke $L_1$ auf den Querschnitt $F_2$ vermindert, der Querschnitt $F_2$ wird auf der Strecke $L_2$ konstant gehalten und anschließend wird der Querschnitt $F_2$ auf der

Strecke $L_3$ wieder auf den Querschnitt $F_1$ geweitet. Dabei ist der Querschnitt $F_1$ größer als der Querschnitt $F_2$ und die Länge $L_3$ größer als die Länge $L_1$.

Die Dosierung der Edukte für die Neutralisation erfolgt vorzugsweise im Bereich der Strecke $L_2$ mit dem Querschnitt $F_2$.

Die optimale Auslegung eines Venturi-Rohrs ist dem Fachmann an sich bekannt. Vorzugsweise wird das Venturi-Rohr so ausgelegt, dass der Druck im Bereich der Strecke $L_2$ weniger als der Umgebungsdruck beträgt (Saugförderung) und/oder das die Strömung im Bereich der Strecke $L_2$ turbulent ist, wobei die Reynolds-Zahl mindestens 1000, vorzugsweise mindestens 2000, besonders bevorzugt mindestens 3000, ganz besonders bevorzugt mindestens 4000, und üblicherweise weniger als 10.000.000 betragen sollte.

Das erfindungsgemäße Neutralisationsverfahren ermöglicht es neutralisierte Lösungen gleichbleibender Qualität bereitzustellen. Das erfindungsgemäße Neutralisationsverfahren eignet sich ganz besonders für kontinuierliche Neutralisationen.

Ebenfalls offenbart ist ein Verfahren zur Herstellung wasserabsorbierender Polymere, in dem eine gemäß dem erfindungsgemäßen Neutralisationsverfahren hergestellte neutralisierte Lösung als Monomerlösung verwendet wird.

Vorzugsweise wird das erfindungsgemäße Neutralisationsverfahren mit einem kontinuierlichen Polymerisationsverfahren kombiniert, wobei vorzugsweise alle Verfahrensschritte, wie Neutralisation, Polymerisieren, Trocknen, Mahlen, Sieben, Nachvernetzen, Sieben, kontinuierlich durchgeführt werden.

Die wasserabsorbierenden Polymere werden beispielsweise durch Polymerisation einer Monomerlösung, enthaltend

a) mindestens eine ethylenisch ungesättigtes Carbonsäure,

b) mindestens einen Vernetzer,

c) wahlweise ein oder mehrere mit dem Monomeren a) copolymerisierbare ethylenisch und/oder allylisch ungesättigte Monomere und

d) wahlweise ein oder mehrere wasserlösliche Polymere, auf die die Monomere a), b) und ggf. c) zumindest teilweise aufgepfropft werden können,

erhalten.

Geeignete ethylenisch ungesättigte Carbonsäuren a) sind beispielseweise Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

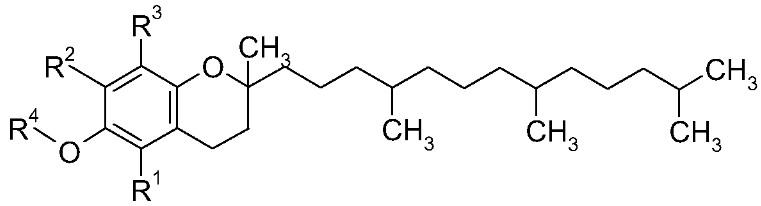

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 0 530 438 beschrieben, Di- und Triacrylate, wie in EP-A 0 547 847, EP-A 0 559 476, EP-A 0 632 068, WO 93/21237,

WO 03/104299, WO 03/104300, WO 03/104301 und DE-A 103 31 450 beschrieben, gemischte Acrylate, die neben Acrylatgruppen weitere ethylenisch ungesättigte Gruppen enthalten, wie in DE-A 103 31 456 und WO 04/013064 beschrieben, oder Vernetzermischungen, wie beispielsweise in DE-A 195 43 368, DE-A 196 46 484, WO 90/15830 und WO 02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 0 343 427 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO 03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Die Menge an Vernetzer b) beträgt vorzugsweise 0,01 bis 1 Gew.-%, besonders bevorzugt 0,05 bis 0,5 Gew.-%, ganz besonders bevorzugt 0,1 bis 0,3 Gew.-%, jeweils bezogen auf das Monomer a).

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Üblicherweise werden die Monomerlösungen vor der Polymerisation weitgehend von Sauerstoff befreit (Inertisierung), beispielsweise mittels Durchströmen mit einem inerten Gas, vorzugsweise Stickstoff. Dadurch werden die Polymerisationsinhibitoren in ihrer Wirkung deutlich abgeschwächt. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten Grundpolymers sowie weitere geeignete hydrophile ethylenisch ungesättigte Monomere d) werden in DE-A 199 41 423, EP-A 0 686 650, WO 01/45758 und WO 03/104300 beschrieben.

Wasserabsorbierende Polymere werden üblicherweise durch Polymerisation einer wässrigen Monomerlösung und wahlweise einer anschließenden Zerkleinerung des Hydrogels erhalten. Geeignete Herstellverfahren sind in der Literatur beschrieben. Wasserabsorbierende Polymere können beispielsweise erhalten werden durch

- Gelpolymerisation im Batchverfahren bzw. Rohrreaktor und anschließender Zerkleinerung im Fleischwolf, Extruder oder Kneter (EP-A 0 445 619, DE-A 198 46 413)
- Polymerisation im Kneter, wobei durch beispielsweise gegenläufige Rührwellen kontinuierlich zerkleinert wird, (WO 01/38402)
- Polymerisation auf dem Band und anschließende Zerkleinerung im Fleischwolf, Extruder oder Kneter (DE-A 38 25 366, US 6,241,928)
- Emulsionspolymerisation, wobei bereits Perlpolymerisate relativ enger Gelgrößenverteilung anfallen (EP-A 0 457 660)
- In-situ Polymerisation einer Gewebeschicht, die zumeist im kontinuierlichen Betrieb zuvor mit wässriger Monomerlösung besprüht und anschließend einer Photopolymerisation unterworfen wurde (WO 02/94328, WO 02/94329)

Die Umsetzung wird vorzugsweise in einem Kneter, wie beispielsweise in WO 01/38402 beschrieben, oder auf einem Bandreaktor, wie beispielsweise in EP-A 0 955 086 beschrieben, durchgeführt.

Die Neutralisation kann auch teilweise nach der Polymerisation auf der Stufe des Hydrogels durchgeführt werden. Es ist daher möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Die Monomerlösung kann durch Einmischen des Neutralisationsmittels neutralisiert werden. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung auf den Endneutralisationsgrad ist bevorzugt.

Das neutralisierte Hydrogel wird dann mit einem Band- oder Walzentrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. 430.2-02 "Moisture content" bestimmt wird. Wahlweise kann zur Trocknung aber auch ein Wirbelbetttrockner oder ein beheizter Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß umso einfacher und das Produkt umso weißer, je höher der Feststoffgehalt des Gels ist. Bevorzugt liegt der Feststoffgehalt des Gels vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Das getrocknete Hydrogel wird vorzugsweise gemahlen und gesiebt, wobei zur Mahlung üblicherweise Walzenstühle, Stiftmühlen oder Schwingmühlen eingesetzt werden können. Die Partikelgröße des gesiebten, trockenen Hydrogels beträgt vorzugsweise unter 1000 $\mu$m, besonders bevorzugt unter 900 $\mu$m, ganz besonders bevorzugt unter 800 $\mu$m, und vorzugsweise über 100 $\mu$m, besonders bevorzugt über 150 $\mu$m, ganz besonders bevorzugt über 200 $\mu$m.

Ganz besonders bevorzugt ist eine Partikelgröße (Siebschnitt) von 106 bis 850 $\mu$m. Die Partikelgröße wird gemäß der von der EDANA (European Disposables and Nonwovens Association) empfohlenen Testmethode Nr. 420.2-02 "Particle size distribution" bestimmt.

Die Grundpolymere werden vorzugsweise anschließend oberflächennachvernetzt. Hierzu geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 0 083 022, EP-A 0 543 303 und EP-A 0 937 736 beschrieben, di- oder polyfunktionelle Alkohole, wie in DE-C 33 14 019, DE-C 35 23 617 und EP-A 0 450 922 beschrieben, oder ß-Hydroxyalkylamide, wie in DE-A 102 04 938 und US 6,239,230 beschrieben.

Des weiteren sind in DE-A 40 20 780 zyklische Karbonate, in DE-A 198 07 502 2-Oxazolidon und dessen Derivate, wie 2-Hydroxyethyl-2-oxazolidon, in DE-A 198 07 992 Bis- und Poly-2-oxazolidinone, in DE-A 198 54 573 2-Oxotetrahydro-1,3-oxazin und dessen Derivate, in DE-A 198 54 574 N-Acyl-2-Oxazolidone, in DE-A 102 04 937 zyklische Harnstoffe, in DE-A 103 34 584 bizyklische Amidacetale, in EP-A 1 199 327 Oxetane und zyklische Harnstoffe und in WO 03/031482 Morpholin-2,3-dion und dessen Derivate als geeignete Oberflächennachvernetzer beschrieben.

Die Nachvernetzung wird üblicherweise so durchgeführt, dass eine Lösung des O-berflächennachvernetzers auf das Hydrogel oder das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt

werden.

Bevorzugte Trocknungstemperaturen liegen im Bereich 50 bis 250°C, bevorzugt bei 50 bis 200°C, und besonders bevorzugt bei 50 bis 150°C. Die bevorzugte Verweilzeit bei dieser Temperatur im Reaktionsmischer oder Trockner beträgt unter 30 Minuten, besonders bevorzugt unter 10 Minuten.

Ein weiterer Gegenstand der vorliegenden Erfindung ist eine Vorrichtung zur Durchführung des erfindungsgemäßen Neutralisationsverfahrens, umfassend

i) eine Ringleitung R,
ii) mindestens eine erste Zuleitung $Z_1$ in die Ringleitung R,
iii) mindestens eine zweite Zuleitung $Z_2$ in die Ringleitung R,
iv) mindestens einen Wärmeaustauscher W in der Ringleitung R wobei sich der Wärmeaustauscher W in Flussrichtung hinter den Zuleitungen $Z_1$ und $Z_2$ befindet,
v) mindestens ein Messgerät $M_1$ zur Bestimmung von Neutralisationsgrad, wobei der Gehalt an Acrylsäure und Natriumacrylat ermittlet wird, und/oder Feststoffgehalt, wobei sich das Messgerät $M_1$ vorzugsweise in Flussrichtung hinter den Zuleitungen $Z_1$ und $Z_2$ und vor dem Wärmeaustauscher W befindet und/oder mindestens ein Messgerät $M_2$ zur Bestimmung des Alkaligehalts, wobei sich das Messgerät $M_2$ in der Zuleitung $Z_1$ oder $Z_2$ befindet,
vi) mindestens eine Ableitung A aus der Ringleitung R, wobei sich die Ableitung A in Flussrichtung hinter dem Wärmeaustauscher W befindet,
vii) eine Pumpe P und
viii) wahlweise einen Behälter B zwischen dem Wärmeaustauscher W und der Ableitung A.

wobei mindestens eine erste Zuleitung $Z_1$ bedeutet, dass das Edukt 1, beispielsweise Natronlauge, über eine oder mehrere Zuleitungen $Z_1$ zugeführt wird, und mindestens eine zweite Zuleitung $Z_2$ bedeutet, dass das Edukt 2, beispielsweise Acrylsäure, über eine oder mehrere Zuleitungen $Z_2$ zugeführt wird.

Das Messgerät $M_1$ ist vorzugsweise ein NIR-Spektrometer.

Das Messgerät $M_2$ ist vorzugsweise eine Kombination aus Dichte- und Temperaturmessung.

Der Ringleitungsquerschnitt Q beträgt von vorzugsweise von 20 bis 2000 $cm^2$ , besonders bevorzugt von 80 bis 700 $cm^2$, ganz besonders bevorzugt von 200 bis 500 $cm^2$. Die Ringleitung R hat vorzugsweise einen kreisrunden Querschnitt.

Die Summe der Zuleitungen $Z_1$ hat einen Querschnitt vorzugsweise von 1,5 bis 100 $cm^2$, besonders bevorzugt von 6 bis 35 $cm^2$, ganz besonders bevorzugt von 15 bis 25 $cm^2$. Die Zuleitungen $Z_1$ haben vorzugsweise einen kreisrunden Querschnitt.

Die Summe der Zuleitungen $Z_2$ hat einen Querschnitt vorzugsweise von 1,5 bis 100 $cm^2$, besonders bevorzugt von 6 bis 35 $cm^2$, ganz besonders bevorzugt von 15 bis 25 $cm^2$. Die Zuleitungen $Z_2$ haben vorzugsweise einen kreisrunden Querschnitt.

Die Pumpe P hat eine Förderleistung von vorzugsweise 1 bis 1000 t/h, besonders bevorzugt von 10 bis 700 t/h, ganz besonders bevorzugt von 100 bis 500 t/h.

Der Behälter B hat ein Volumen von vorzugsweise 1 bis 100 $m^3$, besonders bevorzugt von 10 bis 100 $m^3$, ganz besonders bevorzugt von 20 bis 50 $m^3$.

Die Zuleitungen $Z_1$ und $Z_2$ sind vorzugsweise hintereinander angeordnet, wobei die Zuleitungen $Z_1$ in Flussrichtung vor den Zuleitungen $Z_2$ liegen.

Der Abstand zwischen den Zuleitungen $Z_1$ und $Z_2$ beträgt vorzugsweise 10 bis 500%, besonders bevorzugt 50 bis 300%, ganz besonders bevorzugt 80 bis 200%, der Quadratwurzel des Ringleitungsquerschnitts Q.

Es sind vorzugsweise mindestens zwei Zuleitungen $Z_1$ und/oder $Z_2$ vorhanden, besonders bevorzugt zwei, drei, vier, fünf oder sechs Zuleitungen $Z_1$ bzw. $Z_2$, wobei die Zuleitungen $Z_1$ bzw. $Z_2$ vorzugsweise so angeordnet sind, dass sie eine gemeinsame Achse aufweisen (für zwei Zuleitungen $Z_1$ bzw. $Z_2$) oder einen symmetrischen Stern bilden (für mindestens drei Zuleitungen $Z_1$ bzw. $Z_2$) und die Achse bzw. Stern senkrecht zur Flussrichtung der neutralisierten Lösung befindet (Mehrfachzugabestellen).

Besonders vorteilhaft werden zwei, drei oder vier Mehrfachzugabestellen hintereinander angeordnet.

Beispielsweise können mindestens acht Zuleitungen $Z_1$ vorhanden sein, wobei jeweils vier Zuleitungen $Z_1$ kreuzförmig in die Ringleitung R münden, die mindestens 2 Vierergruppen an Zuleitungen $Z_1$ hintereinander und gegeneinander versetzt angeordnet sind.

Weiterhin kann mindestens eine dritte Zuleitung $Z_3$ in die Ringleitung R münden, wobei mindestens eine dritte Zuleitung $Z_3$ bedeutet, dass das Edukt 3, beispielsweise Wasser, über eine oder mehrere Zuleitungen $Z_3$ zugeführt wird, und die Zuleitung $Z_3$ in Flussrichtung vor der Zuleitung $Z_1$ liegt und/oder die Zuleitung $Z_1$ umhüllt.

Der Abstand zwischen den Zuleitungen $Z_3$ und $Z_1$ beträgt vorzugsweise 10 bis 500%, besonders bevorzugt 50 bis 300%, ganz besonders bevorzugt 80 bis 200%, der Quadratwurzel des Ringleitungsquerschnitts Q.

Vorzugsweise ist die Ringleitung R an mindestens einer Zuleitung $Z_1$ bis $Z_3$ als Venturi-Rohr ausgebildet.

Die Zuleitungen münden besonders bevorzugt alle in ein gemeinsames Venturi-Rohr.

## Patentansprüche

1. Neutralisationsverfahren, wobei mindestens eine ethylenisch ungesättigte Carbonsäure zumindest teilweise mit einer Base neutralisiert wird, die Temperatur der ethylenisch ungesättigten Carbonsäure von 15 bis 40°C beträgt und die ethylenisch ungesättigte Carbonsäure Acrylsäure ist, **dadurch gekennzeichnet, dass** der Gehalt mindestens einer Komponente mindestens eines Stoffstromes der Neutralisation kontinuierlich ermittelt und zur Regelung der Neutralisation genutzt wird.

2. Verfahren gemäß Anspruch 1, **dadurch gekennzeichnet, dass** der Neutralisationsgrad und/oder der Feststoffgehalt der neutralisierten Lösung kontinuierlich bestimmt und über die Dosierung von Base, ethylenisch ungesättigter Carbonsäure und/oder Wasser geregelt wird.

3. Verfahren gemäß Anspruch 2, **dadurch gekennzeichnet, dass** der Neutralisationsgrad und/oder der Feststoffgehalt der neutralisierten Lösung mittels NIR-Spektren bestimmt wird.

4. Verfahren gemäß einem der Ansprüche 1 bis 3, **dadurch gekennzeichnet, dass** die Base wässriges Alkali ist.

5. Verfahren gemäß Anspruch 4, **dadurch gekennzeichnet, dass** der Alkaligehalt des wässrigen Alkali kontinuierlich bestimmt und über die Dosierung von Wasser geregelt wird.

6. Verfahren gemäß Ansprüche 5, **dadurch gekennzeichnet, dass** der Gehalt an wässrigem Alkali mittels einer Messung von Dichte und Temperatur bestimmt wird.

7. Verfahren gemäß einem der Ansprüche 1 bis 6, **dadurch gekennzeichnet, dass** die neutralisierte Lösung teilweise in die Neutralisation rückgeführt wird.

8. Verfahren gemäß Anspruch 7, **dadurch gekennzeichnet, dass** zwischen 25 und 95% der neutralisierten Lösung rückgeführt werden.

9. Verfahren gemäß Anspruch 7 oder 8, **dadurch gekennzeichnet, dass** die rückgeführte neutralisierte Lösung in der Neutralisation nacheinander mit Base und ethylenisch ungesättigter Carbonsäure versetzt wird.

10. Verfahren gemäß Anspruch 7 oder 8, **dadurch gekennzeichnet, dass** die rückgeführte neutralisierte Lösung in der Neutralisation nacheinander mit Wasser, Base und ethylenisch ungesättigter Carbonsäure versetzt wird.

11. Verfahren gemäß einem der Ansprüche 4 bis 10 **dadurch gekennzeichnet, dass** die ethylenisch ungesättigte Carbonsäure eine Temperatur von 15 bis 25°C und/oder das wässrige Alkali einen Alkaligehalt von weniger als 25 Gew.-% und eine Temperatur von 15 bis 30°C oder das wässrige Alkali einen Alkaligehalt von mindestens 25 Gew.-% und eine Temperatur von 30 bis 50°C und/oder falls eingesetzt das Wasser eine Temperatur von 15 bis 30°C aufweist.

12. Verfahren gemäß einem der Ansprüche 1 bis 11, **dadurch gekennzeichnet, dass** mindestens eine Dosierstelle für Base, ethylenisch ungesättigte Carbonsäure und wahlweise Wasser als Venturi-Rohr ausgebildet ist.

13. Verfahren gemäß einem der Ansprüche 4 bis 12, **dadurch gekennzeichnet, dass** das wässrige Alkali Natronlauge ist.

14. Verfahren zur Herstellung wasserabsorbierender Polymere, umfassend ein Neutralisationsverfahren gemäß einem der Ansprüche 1 bis 13.

15. Vorrichtung zur kontinuierlichen Neutralisation, umfassend

i) eine Ringleitung R,
ii) mindestens eine erste Zuleitung $Z_1$ in die Ringleitung R,
iii) mindestens eine zweite Zuleitung $Z_2$ in die Ringleitung R,

iv) mindestens einen Wärmeaustauscher W in der Ringleitung R wobei sich der Wärmeaustauscher W in Flussrichtung hinter den Zuleitungen $Z_1$ und $Z_2$ befindet,
v) mindestens ein Messgerät $M_1$ zur Bestimmung von Neutralisationsgrad, wobei der Gehalt an Acrylsäure und Natriumacrylat ermittelt wird, und/oder Feststoffgehalt, wobei sich das Messgerät $M_1$ vorzugsweise in Flussrichtung hinter den Zuleitungen $Z_1$ und $Z_2$ und vor dem Wärmeaustauscher W befindet und/oder mindestens ein Messgerät $M_2$ zur Bestimmung des Alkaligehalts, wobei sich das Messgerät $M_2$ in der Zuleitung $Z_1$ oder $Z_2$ befindet,
vi) mindestens eine Ableitung A aus der Ringleitung R, wobei sich die Ableitung A in Flussrichtung hinter dem Wärmeaustauscher W befindet,
vii) eine Pumpe P und
viii) wahlweise einen Behälter B zwischen dem Wärmeaustauscher W und der Ableitung A.

**16.** Vorrichtung gemäß Anspruch 15, **dadurch gekennzeichnet, dass** das Messgerät $M_1$ geeignet ist NIR-Messungen durchzuführen.

**17.** Vorrichtung gemäß Anspruch 15, **dadurch gekennzeichnet, dass** das Messgerät $M_2$ geeignet ist die Dichte und die Temperatur zu bestimmen.

## Claims

**1.** A neutralization process in which at least one ethylenically unsaturated carboxylic acid is neutralized at least partly with a base, the temperature of the ethylenically unsaturated carboxylic acid is from 15 to 40°C and the ethylenically unsaturated carboxylic acid is acrylic acid, which comprises determining the content of at least one component of at least one stream of the neutralization continuously and utilizing it for regulating the neutralization.

**2.** The process according to claim 1, wherein the degree of neutralization and/or the solids content of the neutralized solution is determined continuously and is regulated by means of the metered addition of base, ethylenically unsaturated carboxylic acid and/or water.

**3.** The process according to claim 2, wherein the degree of neutralization and/or the solids content of the neutralized solution is determined by means of NIR spectra.

**4.** The process according to any of claims 1 to 3, wherein the base is aqueous alkali.

**5.** The process according to claim 4, wherein the alkali content of the aqueous alkali is determined continuously and is regulated by means of the metered addition of water.

**6.** The process according to claim 5, wherein the content of aqueous alkali is determined by means of a measurement of density and temperature.

**7.** The process according to any of claims 1 to 6, wherein the neutralized solution is recycled partly into the neutralization.

**8.** The process according to claim 7, wherein between 25 and 95% of the neutralized solution is recycled.

**9.** The process according to claim 7 or 8, wherein the recycled neutralized solution, in the neutralization, is admixed successively with base and ethylenically unsaturated carboxylic acid.

**10.** The process according to claim 7 or 8, wherein the recycled neutralized solution, in the neutralization, is admixed successively with water, base and ethylenically unsaturated carboxylic acid.

**11.** The process according to any of claims 4 to 10, wherein the ethylenically unsaturated carboxylic acid has a temperature of from 15 to 25°C and/or the aqueous alkali has an alkali content of less than 25% by weight and a temperature of from 15 to 30°C or the aqueous alkali has an alkali content of at least 25% by weight and a temperature of from 30 to 50°C and/or, if used, the water has a temperature of from 15 to 30°C.

**12.** The process according to any of claims 1 to 11, wherein at least one metering point for base, ethylenically unsaturated carboxylic acid and, if desired, water is designed as a Venturi tube.

**13.** The process according to any of claims 4 to 12, wherein the aqueous alkali is sodium hydroxide solution.

**14.** A process for preparing water-absorbing polymers, comprising a neutralization process according to any of claims 1 to 13.

**15.** An apparatus for continuous neutralization, comprising

i) a ring line R,
ii) at least one first inlet $Z_1$ into the ring line R,
iii) at least one second inlet $Z_2$ into the ring line R,
iv) at least one heat exchanger W in the ring line R, the heat exchanger W being disposed beyond the inlets $Z_1$ and $Z_2$ in flow direction,
v) at least one measuring unit $M_1$ for determining the degree of neutralization, the content of acrylic acid and sodium acrylate being determined, and/or solids content, the measuring unit $M_1$ preferably being disposed beyond the inlets $Z_1$ and $Z_2$ and before the heat exchanger W in flow direction, and/or at least one measuring unit $M_2$ for determining the alkali content, the measuring unit $M_2$ being disposed in the inlet $Z_1$ or $Z_2$,
vi) at least one outlet A from the ring line R, the outlet A being disposed beyond the heat exchanger W in flow direction,
vii) a pump P and
viii) if desired, a vessel B between the heat exchanger W and the outlet A.

**16.** The apparatus according to claim 15, wherein the measuring unit $M_1$ is suitable for carrying out NIR measurements.

**17.** The apparatus according to claim 15, wherein the measuring unit $M_2$ is suitable for determining the density and the temperature.

## Revendications

**1.** Procédé de neutralisation, au moins un acide carboxylique éthyléniquement insaturé étant neutralisé au moins partiellement par une base, la température de l'acide carboxylique éthyléniquement insaturé étant de 15 à 40°C et l'acide carboxylique éthyléniquement insaturé étant de l'acide acrylique, **caractérisé en ce que** la teneur en au moins un composant d'au moins un flux massique de la neutralisation est déterminée en continu et utilisée pour la régulation de la neutralisation.

**2.** Procédé selon la revendication 1, **caractérisé en ce que** le degré de neutralisation et/ou la teneur en solides de la solution neutralisée est/sont déterminé (e) (s) en continu et régulé (e) (s) via le dosage de la base, de l'acide carboxylique éthyléniquement insaturé et/ou d'eau.

**3.** Procédé selon la revendication 2, **caractérisé en ce que** le degré de neutralisation et/ou la teneur en solides de la solution neutralisée est/sont déterminé(e)(s) au moyen de spectres NIR.

**4.** Procédé selon l'une quelconque des revendications 1 à 3, **caractérisé en ce que** la base est un alcali aqueux.

**5.** Procédé selon la revendication 4, **caractérisé en ce que** la teneur alcaline de l'alcali aqueux est déterminée en continu et régulée via le dosage d'eau.

**6.** Procédé selon la revendication 5, **caractérisé en ce que** la teneur en alcali aqueux est déterminée au moyen d'une mesure de la densité et de la température.

**7.** Procédé selon l'une quelconque des revendications 1 à 6, **caractérisé en ce que** la solution neutralisée est recyclée partiellement dans la neutralisation.

**8.** Procédé selon la revendication 7, **caractérisé en ce qu'**on recycle entre 25 et 95% de la solution neutralisée.

**9.** Procédé selon la revendication 7 ou 8, **caractérisé en ce que** la solution neutralisée recyclée est additionnée, dans la neutralisation, consécutivement, de base et d'acide carboxylique éthyléniquement insaturé.

**10.** Procédé selon la revendication 7 ou 8, **caractérisé en ce que** la solution neutralisée recyclée est additionnée, dans la neutralisation, consécutivement, d'eau, de base et d'acide carboxylique éthyléniquement insaturé.

**11.** Procédé selon l'une quelconque des revendications 4 à 10, **caractérisé en ce que** l'acide carboxylique éthyléniquement insaturé présente une température de 15 à 25°C et/ou l'alcali aqueux présente une teneur alcaline inférieure à 25% en poids et une température de 15 à 30°C ou l'alcali aqueux présente une teneur alcaline d'au moins 25% en poids et une température de 30 à 50°C et/ou, si elle est utilisée, l'eau présente une température de 15 à 30°C.

**12.** Procédé selon l'une quelconque des revendications 1 à 11, **caractérisé en ce qu'**au moins un site de dosage pour la base, l'acide carboxylique éthyléniquement insaturé et le cas échéant l'eau est réalisé sous forme de tuyau Venturi.

**13.** Procédé selon l'une quelconque des revendications 4 à 12, **caractérisé en ce que** l'alcali aqueux est de la lessive de soude caustique.

**14.** Procédé pour la préparation de polymères absorbant l'eau, comprenant un procédé de neutralisation selon l'une quelconque des revendications 1 et 13.

**15.** Dispositif pour la neutralisation continue, comprenant

i) une conduite annulaire R,
ii) au moins une première conduite d'alimentation $Z_1$ dans la conduite annulaire R,
iii) au moins une deuxième conduite d'alimentation $Z_2$ dans la conduite annulaire R,
iv) au moins un échangeur thermique W dans la conduite annulaire R, l'échangeur thermique W se trouvant, dans le sens d'écoulement, en aval des conduites d'alimentation $Z_1$ et $Z_2$,
v) au moins un appareil de mesure $M_1$ pour la détermination du degré de neutralisation, la teneur en acide acrylique et en acrylate de sodium étant déterminée et/ou la teneur en solides, l'appareil de mesure $M_1$ se trouvant de préférence, dans le sens d'écoulement, en aval des conduites d'alimentation $Z_1$ et $Z_2$ et en amont de l'échangeur thermique W et/ou au moins un appareil de mesure $M_2$ pour la détermination de la teneur alcaline, l'appareil de mesure $M_2$ se trouvant dans la conduite d'alimentation $Z_1$ ou $Z_2$,
vi) au moins une dérivation A de la conduite annulaire R, la dérivation A se trouvant, dans le sens d'écoulement, en aval de l'échangeur thermique W,
vii) une pompe P et
viii) éventuellement un récipient B entre l'échangeur thermique W et la dérivation A.

**16.** Dispositif selon la revendication 15, **caractérisé en ce que** l'appareil de mesure $M_1$ convient pour réaliser des mesures NIR.

**17.** Dispositif selon la revendication 15, **caractérisé en ce que** l'appareil de mesure $M_2$ convient pour déterminer la densité et la température.

Figur 1

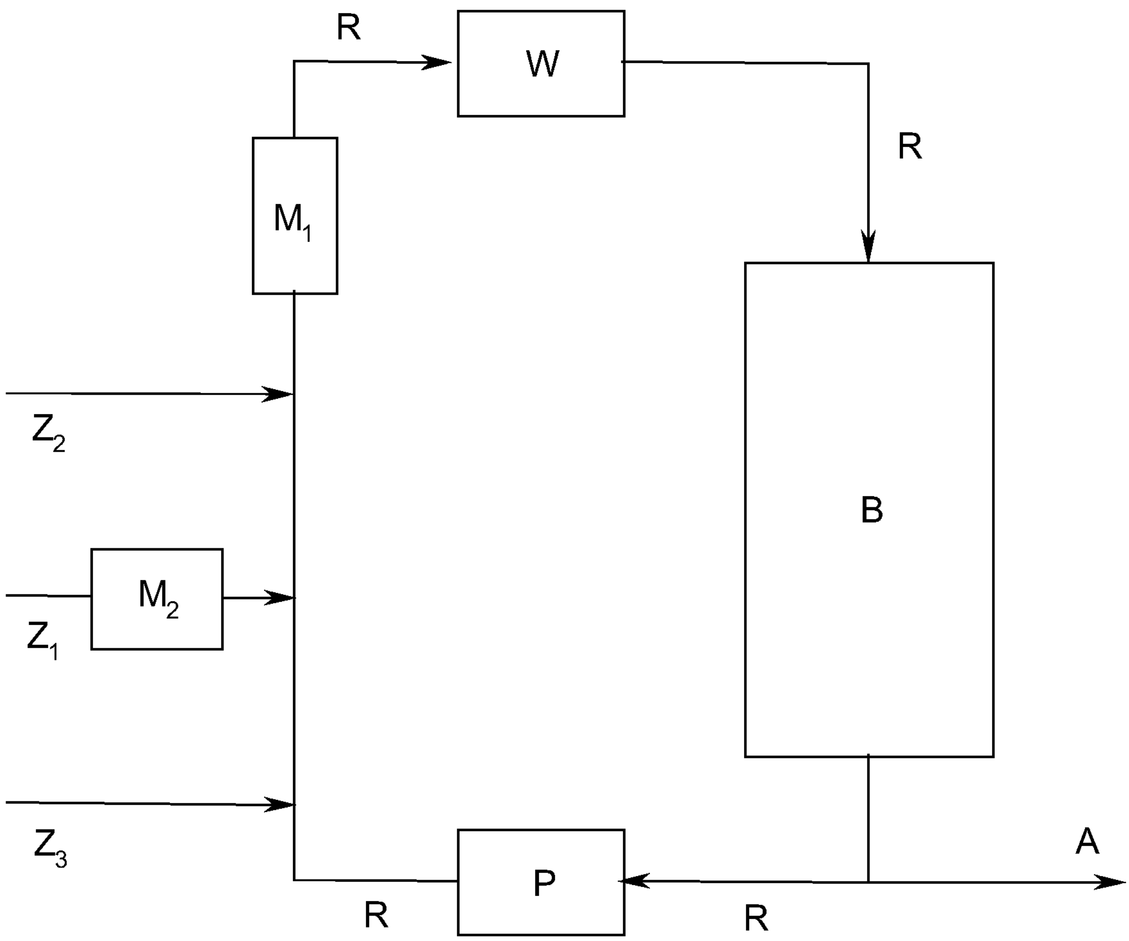

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0138402 A
- WO 03004237 A
- WO 03022896 A
- WO 01016197 A
- EP 0372706 A
- EP 0574260 A
- WO 03051415 A
- WO 03095410 A1
- EP 0530438 A
- EP 0547847 A
- EP 0559476 A
- EP 0632068 A
- WO 9321237 A
- WO 03104299 A
- WO 03104300 A
- WO 03104301 A
- DE 10331450 A
- DE 10331456 A
- WO 04013064 A
- DE 19543368 A
- DE 19646484 A
- WO 9015830 A
- WO 0232962 A
- EP 0343427 A
- DE 19941423 A
- EP 0686650 A
- WO 0145758 A
- EP 0445619 A
- DE 19846413 A
- DE 3825366 A
- US 6241928 B
- EP 0457660 A
- WO 0294328 A
- WO 0294329 A
- EP 0955086 A
- EP 0083022 A
- EP 0543303 A
- EP 0937736 A
- DE 3314019 C
- DE 3523617 C
- EP 0450922 A
- DE 10204938 A
- US 6239230 B
- DE 4020780 A
- DE 198075022 A
- DE 19807992 A
- DE 198545732 A
- DE 10204937 A
- DE 10334584 A
- EP 1199327 A
- WO 03031482 A


### In der Beschreibung aufgeführte Nicht-Patentliteratur


- Modern Superabsorbent Polymer Technology. **F.L. BUCHHOLZ ; A.T. GRAHAM.** Monographie. Wiley-VCH, 1998
- Ullmann's Encyclopedia of Industrial Chemistry. vol. 35, 73-103
- Ullmann's Encyclopedia of Industrial Chemistry. vol. 35, 35